(19) Europäisches Patentamt
European Patent Office
Office européen des brevets

(11) Publication number: **0 162 239 B1**

# (12) EUROPEAN PATENT SPECIFICATION

(45) Date of publication of patent specification: **07.08.91** (51) Int. Cl.[5]: **A61K 47/00**

(21) Application number: **85103811.7**

(22) Date of filing: **29.03.85**

## (54) Percutaneous absorption accelerator and preparation containing same.

(30) Priority: **23.04.84 JP 82464/84**
**19.07.84 JP 150154/84**

(43) Date of publication of application:
**27.11.85 Bulletin 85/48**

(45) Publication of the grant of the patent:
**07.08.91 Bulletin 91/32**

(84) Designated Contracting States:
**CH DE FR GB IT LI NL**

(56) References cited:
**EP-A- 0 035 132**
**EP-A- 0 144 069**

**CHEMICAL ABSTRACTS, vol. 97, no. 24, 13th December 1982, page 323, no. 203091f, Columbus, Ohio, US; & JP - A - 82 123 108 (KAO SOA CO., LTD.) 31-07-1982**

(73) Proprietor: **Kao Corporation**
**14-10, Nihonbashi Kayabacho 1-chome**
**Chuo-Ku Tokyo 103(JP)**

(72) Inventor: **Hara, Kenji**
**1022-53, Himuro-machi**
**Utsunomiya-shi, Tochigi-ken(JP)**
Inventor: **Kamiya, Tetsuro**
**2606-6, Akabane Ichikai-machi**
**Haga-gun Tochigi-ken(JP)**
Inventor: **Inoue, Takeshi**
**2071, Yanase-machi Utsunomiya-shi**
**Tochigi-ken(JP)**
Inventor: **Yorozu, Hidenori**
**1729, Mashiko Mashiko-machi**
**Haga-gun Tochigi-ken(JP)**
Inventor: **Eguchi, Yasuteru**
**513-6, Mine-machi Utsunomiya-shi**
**Tochigi-ken(JP)**
Inventor: **Tsujii, Kaoru**
**1022-88, Himuro-machi Utsunomiya-shi**
**Tochigi-ken(JP)**

(74) Representative: **Kraus, Walter, Dr. et al**
**Patentanwälte Kraus, Weisert & Partner**
**Thomas-Wimmer-Ring 15**
**W-8000 München 22(DE)**



Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to a percutaneous absorption accelerator and a percutaneous absorbent preparation containing the same and, more particularly to a percutaneous absorption accelerator containing ether derivatives of specific glycerols or polyglycerols and alcohols as effective components and, percutaneous absorbent preparation containing the percutaneous absorption accelerators and pharmaceutically effective components.

As methods for administration of drugs, oral administration, rectal administration and intracutaneous administration, have been hitherto generally adopted. Among them, oral administration has been widely employed. However, in the case of oral administration, the oral administration encountered defects that side effects such as gastrointestinal disturbance, anorexia, vomitting and abdominal pain, have been caused sometimes and, at the same time, it is necessary, in most cases, to administer large quantities of drugs. In recent years, preparations through percutaneous administration have been developed for purposes of eliminating such defects, expecting that the side effects would be minimized and pharmacological effects would be exhibited more safely and, some products have been commercially available. In many cases, however, percutaneous absorbability of pharmaceutically effective components in such preparations are unsatisfactory yet but the purposes are satisfactorily achieved only with difficulty. Namely, skin, inter alia, its keratin layer which constitutes the outermost layer, functions physiologically and acts as a protective wall against permeation of substances into the body and, in many cases, it is difficult for a base alone used for conventional topical agents to attain percutaneous absorption sufficient for pharmaceutically effective components formulated therein. For this reason, any device is necessary to control the permeability of drugs through keratin layer and enhance percutaneous absorption of drugs.

In addition, for accelerating absorption of pharmacologically active substances from the mucosal portions of the human body - mucous membrane of the eye, nasal mucous membrane, bucal mucos membrane, vaginal mucous membrane and rectal mucous membrane, improvement of preparation form, improvement of bases and formulation of compounds having absorption acceleration effect, have been made. Among them, the improvement of preparation form and the improvement of bases are possible to a certain extent; however, epoch-making improvement is not expectable and, the target of the research has been focused upon search and application of compounds having absorption accelerating effect.

For such purposes, it has been generally done to formulate a so-called percutaneous absorption accelerator in a base. As such absorption accelerators, there are known dimethyl sulfoxide; amide compounds such as dimethyl acetamide, dimethylformamide and N,N-diethyl-m-toluamide; azacycloalkan-2-one derivatives such as 1-dodecylazacyclo-heptan-2-one; esters of alcohols and carboxylic acids such as isopropyl myristate, isopropyl palmitate, diethyl sebacate, diisopropyl adipate; and crotonyl-N-ethyl-o-toluidine. However, these absorption accelerators are still unsatisfactory with their absorption accelerating effect and, in many cases, practically pharmacological effects cannot be obtained. In addition, these absorption accelerators involve such problems in practical use under actual circumstances that the absorption accelerators themselves show irritation to the skin and corrode synthetic resins due to the property as potent solvents to dissolve irritative substances and sensitized substances, out of containers for drugs, clothes and accessories, so that general adoption and use are restricted. Chemical Abstracts, Volume 97, page 323, No. 203091f describes a pharmaceutical ointment base containing $ROCH_2CH(OH)-CH_2OH$ with $R = C_{10-24}$ alkyl or alkenyl.

EP-A-0 035 132 describes pharmaceutical preparations which contain glycerine-1,2-dimethyl-ether.

It now has been found that by formulating specific ether derivatives of glycerols or polyglycerols as percutaneous absorption accelerators in a base, percutaneous absorption of pharmaceutically effective components can be markedly increased and pharmacological effects of the pharmaceutically effective components can be exhibited effectively and safely and thus have accomplished the present invention.

The present invention provides percutaneous absorption accelerators comprising an ether compound of a glycerol or a polyglycerol and an alcohol represented by the following formula (I) and (II):

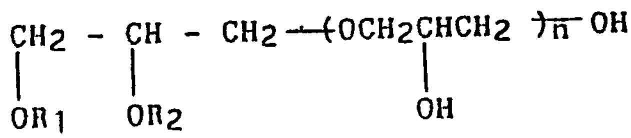

(I)

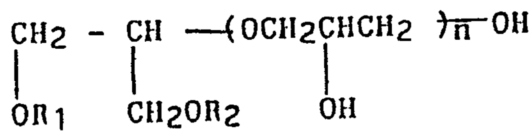

(II)

wherein $R_1$ represents a saturated or unsaturated straight or branched aliphatic hydrocarbon group having 8 to 24 carbon atoms and $R_2$ represents an alkyl group having 1 to 4 carbon atoms and n represents an integer of 1 to 10, as essential components. The invention also provides percutaneous absorption preparations comprising a pharmaceutically effective component. and an ether compound as defined above. The term percutaneous absorption in the present invention means the absorption through a mucosal portion of a human body such as mucous membrane of the eye, nasal mucous membrane, buccal mucous membrane, vaginal mucous membrane and rectal mucous membrane in addition to the topical percutaneous absorption through keratin layer of the skin.

The ether derivatives used in the present invention are obtained by reacting alcohols with glycerols or polyglycerols derived therefrom in a conventional manner. Specific examples of alcohols which are used include straight chain type aliphatic alcohols such as methyl alcohol, ethyl alcohol, propyl alcohol, butyl alcohol, octyl alcohol, decyl alcohol, dodecyl alcohol, hexadecyl alcohol, octadecyl alcohol, and octadecenyl (oleyl) alcohol; branched type aliphatic primary alcohols such as isopropyl alcohol, isobutyl alcohol, 2-ethylhexyl alcohol, 2-hexyldecyl alcohol, 2-octyldodecyl alcohol, 2-heptylundecyl alcohol, 2-(1,3,3-trimethylbutyl)octyl alcohol, 2-decyltetradecyl alcohol, 2-dodecylhexadecyl alcohol, 2-tetradecyloctadecyl alcohol, 5,7,7-trimethyl-2-(1,3,3-trimethylbutyl)octyl alcohol and methyl-branched isostearyl alcohols represented by the following formula:

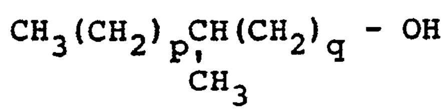

wherein p represents an integer of 4 to 10; and q represents an integer of 5 to 11, wherein p + q represents 11 to 17 and has a distribution which has the peak when p is 7 and q is 8; secondary alcohols such as sec-octyl alcohol, sec-decyl alcohol and sec-dodecyl alcohol; tertiary alcohols such as t-octyl alcohol and.t-docecyl alcohol; alicyclic alcohols such as cylcohexyl alcohol and cyclopentyl alcohol; alkylphenols such as octylphenol and nonylphenol.

The ether derivatives used in the present invention are those represented by the following formula (I) or (II):

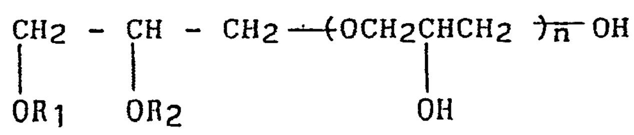

(I)

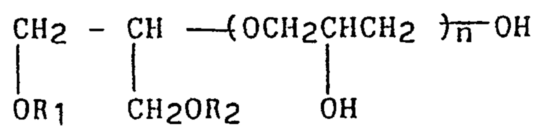

(II)

wherein $R_1$ represents a saturated or unsaturated straight or branched aliphatic hydrocarbon group having 8 to 24 carbon atoms and $R_2$ represents an alkyl group having 1 to 4 carbon atoms and n represents an integer of 1 to 10.

Of the ether derivates (I) or (II), preferred examples include 1-O-alkylglycerols such as 1-O-alkyl-3-O-2',3'-dihydroxypropylglycerols, such as 1-O-n-octyl-3-O-2',3'-dihydroxypropylglycerol, 1-O-n-dodecyl-3-O-2',3'-dihydroxypropylglycerol, l-O-n-tetradecyl-3-O-2',3'-dihydroxypropylglycerol, l-O-n-hexadecyl-3-O-2',3'-dihydroxypropylglycerol, l-O-n-octadecyl-3-O-2',3'-dihydroxypropylglycerol, l-O-n-octadecenyl-3-O-2',3'-dihydroxypropylglycerol and l-O-methyl-branched isostearyl-3-O-2',3'-dihydroxypropylglycerol; l,2-di-O-alkyl-3-O-2',3'-dihydroxypropylglycerols such as l-O-n-octyl-2-O-methyl-3-O-2',3'-dihydroxypropylglycerol,l-O-n-docecyl-2-O-methyl-3-O-2',3'-dihydroxypropylglycerol, l-O-n-dodecyl-2-O-n-butyl-3-O-2',3'-dihydroxypropylglycerol, l-O-n-dodecyl-2-O-n-octyl-3-O-2',3'-dihydroxypropylglycerol, l-O-n-tetradecyl-2-O-methyl-3-O-2',3'-dihydroxypropylglycerol, l-O-n-hexadecyl-2-O-methyl-3-O-2',3'-dihydroxypropylglycerol, l-O-n-octadecyl-2-O-methyl-3-O-2',3'-dihydroxypropylglycerol, l-O-n-octadecenyl-2-O-methyl-3-O-2',3'-dihydroxypropylglycerol, l-O-n-octadecenyl-2-O-n-butyl-3-O-2',3'-dihydroxypropylglycerol, l-O-methyl-branched isostearyl-2-O-methyl-3-O-2',3'-dihydroxypropylglycerol, l-O-methyl-branched isostearyl-2-O-n-octyl-3-O-2',3'-dihydroxypropylglycerol, etc.; l,3-di-O-alkyl-2-O-2',3'-dihydroxypropylglycerols such as l-O-n-octyl-3-O-methyl-2-O-2',3'-dihydroxypropylglycerol,l-O-n-dodecyl-3-O-methyl-2-O-2',3'-dihydroxypropylglycerol, l-O-n-dodecyl-3-O-n-butyl-2-O-2',3'-dihydroxypropylglycerol, l-O-n-dodecyl-3-O-n-octyl-2-O-2',3'-dihydroxypropylglycerol, l-O-n-tetradecyl-3-O-methyl-2-O-2',3'-dihydroxypropylglycerol, l-O-n-hexadecyl-3-O-methyl-2-O-2',3'-dihydroxypropylglycerol, l-O-n-octadecyl-3-O-methyl-2-O-2',3'-dihydroxypropylglycerol, l-O-n-octadecenyl-3-O-methyl-2-O-2',3'-dihydroxypropylglycerol, l-O-n-octadecenyl-3-O-n-butyl-2-O-2',3'-dihydroxypropylglycerol, l-O-methyl-branched isostearyl-3-O-methyl-2-O-2',3'-dihydroxypropylglycerol l-O-methyl-branched isostearyl-3-O-n-butyl-2-O-2',3'-dihydroxypropylglycerol and l-O-methyl-branched isostearyl-3-O-n-octyl-2-O-2',3'-dihydroxypropylglycerol. The ether derivatives of glycerol or polyglycerol with these alcohols have extremely low toxicity in $LD_{50}$ of 5000 mg/kg or more.

The percutaneous absorption accelerator of the present invention is prepared using the ether derivative of glycerol or polyglycerol with alcohols as it is, or by dissolving, dispersing or suspending the ether derivative of glycerol or polyglycerol with alcohols in a suitable solvent such as water, ethanol, propylene glycol and triacetin. Further, the percutaneous absorption accelerator of the present invention may be formulated, if necessary, with known compounds having percutaneously absorbing property, for example, dimethylsulfoxide, dimethylacetamide, dimethylformamide, N,N-dimethyl-m-toluamide and azacycloalkan-2-one derivatives such as l-dodecylazacycloheptan-2-one, esters of alcohols and carboxylic acids such as isopropyl myristate, isopropyl palmitate, diethyl sebacate, diisopropyl adipate and crotonyl-N-ethyl-o-toluidine.

The percutaneous absorbent preparation of the present invention may be formulated by incorporating various pharmaceutically effective components with the percutaneous absorption accelarator. Such percutaneous absorption accelerator can be advantageously used for many preparations for topical agents which are expected to exhibit the pharmacological effect by applying the same to skin, hair and nail to be absorbed therefrom, for example, a liquid spraying agent, a lotion, an ointment, a cream, a gel, a sol, an aerosol, a cataplasm, a plaster and a tape preparation.

Further, in the application as transmucosal administration, the percutaneous absorbent preparation of the present invention is prepared using the above-mentioned transmucosal absorption accelerator either as

it is, or, by formulating the same in various forms of prepartions for transmucosal administration, for example, suppositories for rectal and vaginal administration, an ointment, a soft gelatin capsule, a buccal tablet, a perlingual tablet, a nose drop, or a spraying agent for nasal mucous membrane or buccal mucous membrane, and, if necessary, further adding desired carriers or vehicles for preparations and making preparations in a conventional manner. In addition, the prepartion for transmucosal administration of the present invention may be formulated, if necessary, with known compounds having transmucosal absorbing activity, for example, ether type non-ionic surfactants, enamine derivatives of phenylglycine, N-acylcollagen peptides, sodium salts of medium chain fatty acids and saponins.

It is preferred that the percutaneous absorption accelerator of the present invention be formulated, as the effective component, in an amount of 0.001 to 10% by weight, particularly 0.1 to 8% by weight, into the preparation for percutaneous administration generally used, based on the total amount of the preparation, as an aid for percutaneous absorption. Further in the case of using the percutaneous absorption accelerator as the base for percutaneous absorption, it is also possible to formulate the same in an amount of 10% by weight or more.

Examples in which pharmaceutical effects increase by the utilization of the percutaneous absorvent preparation of the present invention as topical agents include steroid anti-inflammatory agents such as prednisolone, dexamethason, etc.; non-steroid anti-inflammatory agents such as indometacin, fulfenamic acid and mefenamic acid; anti-histamic agents such as triperenamine, insaibenzyl, chlorpheniramine, diphenhydramine and promethazine; sulfa agents such as sulfamonomethoxine and sulfamethizole; antibiotics such as penicillin, cephalosporin, erythromycin, tetracyclin, chloramphenicol and streptomycin; anti-fungal agents such as naphthiomate and clotrimazole; anti-malignant tumor agents such as 5-fluorouracil, cyclophosphamide, busulfan and actinomycin; analgesics such as morphine, codeine, nalorphine pentazocine, aspirin, acetanilide and aminopyrine; preparations of prostaglandins; hypnotics and tranquilizers such as barbital and thiopental; psychotropic agents such as chlorpromazine, reserpine and chlordiazepoxide; anti-epileptic agents; anti-Parkinson's syndrome agents such as chlorzoxazone and revodopa; cardiotonic agents such as digitoxin and digoxin; anti-arrhythmic agents such as procainamide hydrochloride and propanolol hydrochloride; anti-angina pectoris agents such as dipyridamole and amyl nitrite; anti-hypertension agents such as reserpine and guanethidine sulfate; UV inhibitors such as p-aminobenzoate esters; agents for preventing the formation of melanine such as hydroquinone, vitamin C esters and p-hydroxycinnamate; PUVA treating agents against psoriasis such as 8-methoxypsoralen; vitamins such as vitamin A, vitamin E and vitamin C; hormone agents such as insulin, estradiol and methyltestosterone; diagnostics; allergens for patch test; vermicides; insecticides; moisturizers; keratin softening agents and hair dyes;

Further the percutaneous absorbent preparation for the topical agent of the present invention is also effective for many drugs, agricultural chemicals and growth hormones for which pharmacological effects are expected by applying the same to animals, insects and plants to be absorbed therein.

Examples in which pharmaceutical effects increase by the utilization of the transmucosal absorption of the present invention include pharmacologically active polysaccharide substances such as heparin, dextran sulfate, pentosan sulfate (heparinoid) and chondroitin sulfate and salts thereof; glucoamylase inhibitor; peptide type anti-tumor substances such as bleomycin, neocarzinostan and L-asparginase; enzyme preparations such as trypsin, chemotrypsin, bromelain, papain, protenase, peroxidase, nagase, proctase, serratiopeptidase, seaprose, lysozyme, plasmin, urokinase, cytochrome C, hyaluronidase, fibrinolysine, thrombin, callidin, callikrein, plasmin, glucose oxidase, β-galactosidase, fytin, desoxyribonuclease, choline esterase, pronase and pancreatin; peptide hormones such as calcitonin, parathormone, relaxin, insulin, glucagon, prolactin, adrenocorticotropin (ACTH), gonadotropic hormone, thyrotropin (TSH), growth hormone (BGH), luteinizing hormone (LH), follicle-stimulating hormone (FSH), oxytocin, vasopressin, antidiuretic hormone, coherin, melanocyte-stimulating hormone (MSH), gastrin, tetragastrin, pentagastrin, secretin, pancreozymin, cholecystokinin, Substance P, gonadotropin (HCG) and vasopressin; inhibitors for peptide hormone releasing factors such as adrenocorticotropic hormone releasing factor (ACTH-RH), follicle-stimulating hormone releasing factor (FSH-RH), growth hormone releasing factor (GH-IH), luteinizing hormone releasing factor (LH-RH), prolactin releasing factor (PR-RH), prolactin inhibiting factor (PR-IH) and thyroid-stimulating hormone releasing factor (TSH-RH); polynucleotides such as polyribonucleotide, complex of polyinocinic acid and cytidylic acid, complex of polyadenylic acid and polyuridilic acid and polydeoxyribonucleotide; insulin secretion-activating protein (IAP), pancreas-basic trypsin inhibitor, antipain hydrochloride, chymostatin A, elastatinal, pepstatin A, polylysine, polyornithine, polyethylenimine and polyvinylamine; steroid anti-inflammatory agents such as prednisolone and dexamethason; non-steroid anti-inflammatory agents such as indometacin, fulfenamic acid and mefenamic acid; anti-histamic agents such as triperenamine, insaibenzyl, chlorpheniramine, diphenhydramine and promethazine; sulfa agents such as

sulfamonomethoxine and sulfamethizole; antibiotics such as penicilline, cephalosporin, erythromycin, tetracyclin, chloramphenicol and streptomycin; anti-malignant tumor agents such as 5-fluorouracil, cyclophosphamide, busulfan, and actinomycin; analgesics such as morphine, codeine, nalorphine, pentazocine, aspirin, acetanilide and aminopyrine; preparations of prostaglandins; hypnotics and tranquilizers such as barbital and thiopental; psychotropic agents such as chlorpromazine, reserpine and chlordiazepoxide; anti-epileptic agents, anti-Parkinson's syndrome agents such as chlorzoxazone and revodopa; cardiotonic agents such as digitoxin and digoxin; antiarrhythmic agents such as procainamide hydrochloride and propanolol hydrochloride; anti-angina pectoris agents such as dipyridamole and amyl nitrite; anti-hypertension agents such as reserpine and guanethidine sulfate.

In the present percutaneous absorbent preparation, such pharmaceutically effective component may be incorporated in the pharmaceutically effective amount according to particular object to apply such preparation.

In the ether derivative used in the present invention, the structure can be appropriately chosen to control the balance between hydrophilic property and oleophilic property so that it is possible to prepare the ether derivative in any base having either hydrophilic property or oleophilic property. As a result, the ether derivative having high solubility for various pharmaceutically effective components can be chosen according to the present invention. It is thus possible to design topical agents having good handling and high percutaneous absorption by dissolving difficultly soluble pharmaceutically effective components in hydrophilic bases in a high concentration.

Next, the present invention will be concretely described with reference to the examples below but is not deemed to be limited only to these examples.

Test Example 1

Japanese white male rabbits weighing about 2.5 kg, fasted for 24 hours, were fixed at the back and, each of solutions shown in Table 1 was administered to the rectum of about 2.5 cm from the anus using a tube. Cannule was inserted into the femoral vein of the hind leg to collect about 0.2 ml each of blood in every fixed tide. Blood sugar level was measured using destrostick. Change in blood sugar level was determined with the passage of time, as the blood sugar level prior to administration being rendered 100%. The results are shown in Table 1.

Table 1

| | Specimen | | Rate of Change in Blood Sugar Level before Administration (%) 15 mins. | 30 mins. | 45 mins. | 60 mins. | 90 mins. | 120 mins. |
|---|---|---|---|---|---|---|---|---|
| Control: | Physiological saline<br>Ethanol<br>Insulin | 0.75 g<br>0.25 g<br>10/kg | +10.2 | +12.5 | +11.2 | +11.2 | +12.2 | +18.0 |
| | 25% Ethanol solution | | + 5.0 | +12.9 | +11.5 | + 4.8 | + 1.5 | + 2.7 |
| | 1-O-n-Dodecyl-3-O-methyl-2-O-2',3'-dihydroxy-propylglycerol | 1 g | + 6.5 | +10.2 | + 9.8 | + 9.5 | + 9.5 | +10.2 |
| This Inven-tion | Physiological saline<br>Ethanol<br>Insulin<br>1-O-n-Dodecyl-3-O-methyl-2-O-2',3'-dihydroxy-propylglycerol | 0.70 g<br>0.25 g<br>10/kg<br>0.05 g | -13.4 | -18.6 | -25.2 | -27.6 | -25.8 | -22.4 |
| | Physiological saline<br>Ethanol<br>Insulin<br>1-O-n-Dodecyl-3-O-methyl-2-O-2',3'-dihydroxypropyl-glycerol | 0.65 g<br>0.25 g<br>10/kg<br>0.1 g | -19.2 | -28.9 | -32.7 | -28.0 | -19.5 | -18.2 |
| | Physiological saline<br>Ethanol<br>Insulin<br>1-O-Methyl-branched iso-stearyl-3-O-methyl-2-O-2',3'-dihydroxypropyl-glycerol | 0.65 g<br>0.25 g<br>10/kg<br>0.1 g | -17.8 | -18.3 | -24.5 | -28.6 | -21.9 | -19.8 |

Test Example 2

To a mixture of 1.4 g of l-O-n-octyl-3-O-methyl-2-O-2',3'-dihydroxypropylglycerol and 4.5 g of ethanol was added a 17 U/ml insulin solution in physiological saline and, the mixture was made 10 g in total to

prepare a preparation for nasal spraying. The preparation was administered to the nasal cavity of male rabbits, weighing about 2.5 kg, fasted for 24 hours and fixed at the back, at a dose of 1 U/rabbit. Insulin in serum was quantitatively determined by enzyme immunoassay. For control, a preparation obtained by supplementing physiological saline for the above-mentioned l-O-n-octyl-3-O-methyl-2-O-2',3'-dihydroxypropylglycerol was used. The results are shown in Table 2.

Table 2

Concentration of Insulin in Serum (μU/ml)

| | 0 | 20 mins. | 40 mins. | 60 mins. | 90 mins. | 120 mins. | 180 mins. |
|---|---|---|---|---|---|---|---|
| Control | 5 | 8 | 12 | 12 | 12 | 14 | 14 |
| This invention | 7 | 261 | 227 | 95 | 78 | 31 | 28 |

Examle 1

Topical agents containing indometacin shown below were prepared and percutaneous absorption was examined with the topical agents. The results are shown in Table 3.

Preparation

Preparation 1 of the present invention:

An ointment obtained by incorporating 3 g of l-O-n-dodecyl-3-O-methyl-2-O-2',3'-dihydroxypropylglycerol into 97 g of a commercially available gel-like topical agent containing 1 wt% of indometacin, Inteban ointment (made by Sumitomo Chemical Industry Co., Ltd.).

Preparation 2 of the present invention:

An ointment obtained by incorporating 3 g of l-O-methyl-branched isostearyl-3-O-methyl-2-O-2',3'-dihydroxypropylglycerol into 97 g of a commercially available gel-like topical agent containing 1 wt% of indometacin, Inteban ointment (made by Sumitomo Chemical Industry Co., Ltd.).

Preparation 3 of the present invention:

An ointment obtained by incorporating 3 g of l-O-methyl-branched isostearyl-3-O-n-butyl-2-O-2',3'-dihydroxypropylglycerol into 97 g of a commercially available gel-like topical agent containing 1 wt% of indometacin, Inteban ointment (made by Sumitomo Chemical Industry Co., Ltd.).

Preparation 4 of the present invention:

An ointment obtained by incorporating 3 g of l-O-n-octyl-3-O-methyl-2-O-2',3'-dihydroxypropylglycerol into 97 g of a commercially available gel-like topical agent containing 1 wt% of indometacin, Inteban ointment (made by Sumitomo Chemical Industry Co., Ltd.).

Preparation 5 of the present invention:

A liquid topical agent obtained by adding purified water to a mixture of 1 g of indometacin, 14 g of l-O-n-dodecyl-3-O-methyl-2-O-2',3'-dihydroxypropylglycerol and 45 g of ethanol to make 100 g.

Preparation 6 of the present invention:

A liquid topical agent obtained by adding purified water to a mixture of 1 g of indometacin, 14 g of l-O-methyl-branched isostearyl-3-O-methyl-2-O-2',3'-dihydroxypropylglycerol and 45 g of ethanol to make 100 g.

Preparation 7 of the present invention:

A liquid topical agent obtained by adding purified water to a mixture of 1 g of indometacin, 14 g of l-O-methyl-branched isostearyl-3-O-n-butyl-2-O-2',3'-dihydroxypropylglycerol and 45 g of ethanol to make 100 g.

Preparation 8 of the present invention:

A liquid topical agent obtained by adding purified water to a mixture of 1 g of indometacin, 14 g of l-O-n-octyl-3-O-methyl-2-O-2',3'-dihydroxypropylglycerol and 45 g of ethanol to make 100 g.

Comparative preparation 1

A commercially available gel-like topical agent containing 1 wt% of indometacin, Inteban® ointment (made by Sumitomo Chemical Industry Co., Ltd.).

Comparative preparation 2

A liquid topical agent obtained by adding purified water to a mixture of 1 g of indometacin, 14 g of N,N-diethyl-m-toluamide and 45 g of ethanol to make 100 g.

Comparative preparation 3:

A liquid topical agent obtained by adding purified water to a mixture of 1 g of indometacin, 14 g of dimethylsulfoxide and 45 g of ethanol to make 100 g.

Method:

Test of percutaneous absorption of indometacin:

Seven (7) Japanese white female rabbits weighing about 3 kg were used as one group. The topical agents of the present invention and the comparative preparations were applied onto the normal abdominal skin (10 cm x 14 cm) of the rabbits in each group, from which the body hair was cut, respectively, at a dose corresponding to 20 mg of indometacin. Blood was collected from the vein of the ear after 4, 10 and 20 hours and, blood concentration of indometacin was measured.

Table 3

| Preparation | | Maximum Concentration in Serum (Cmax; ng/ml) |
|---|---|---|
| This invention | 1 | 540 |
| " | 2 | 510 |
| " | 3 | 500 |
| " | 4 | 420 |
| Comparative Preparation | 1 | 95 |
| This invention | 5 | 1900 |
| " | 6 | 1750 |
| " | 7 | 1550 |
| " | 8 | 1230 |
| Comparative Preparation | 2 | 190 |
| " | 3 | 150 |

As is clear from the results described above, the topical agents 1 to 8 of the present invention all showed extremely high percutaneous absorption of indometacin as compared to the comparative preparations. In particular, the topical agent of the present invention obtained by formulating l-O-n-dodecyl-3-O-methyl-2-O-2',3'-dihydroxypropylglycerol as a percutaneous absorption accelerator showed extremely high percutaneous absorption of indometacin.

Example 2

With respect to the topical agents of the present invention, the pharmacological effect was examined according to the inhibition of carrageenin caused edema on the paw of the rat. The results are shown in Table 4. Method:

Wistar male 10 rats, weighing about 110 g, were used as one group. The volume of the right rear paw of the rats in each group was previously measured using a branched glass container. A 1% carrageenin aqueous solution was subcutaneously injected to the right rear paw sole in a dose of 0.125 ml. Immediately thereafter 0.3 g of indometacin topical agents were applied to the skin of the right rear paw sole. In the control group, carrageenin alone was injected. Then, the volume of the rear paw was measured every 90 minutes, which was continued until 6 hours after. The rate of edema and the inhibition rate of edema were calculated as described below.

$$\frac{\text{rate of edema (\%)}}{100} = \frac{\text{volume of paw after injection}}{\text{volume of paw before injection}} - 1$$

$$\frac{\text{Inhibition rate of edema (\%)}}{100}$$

$$= 1 - \frac{\text{rate of edema in the treated group}}{\text{rate of edema in the control group}}$$

Table 4

| Preparation | | Inhibition Rate of Edema (%) 1.5 hr | 3.0 hrs | 4.5 hrs | 6.0 hrs |
|---|---|---|---|---|---|
| This invention | 5 | 52.6 | 52.7 | 53.7 | 56.0 |
| " | 8 | 23.7 | 37.4 | 40.8 | 50.3 |
| Comparative Preparation | 1 | 10.5 | 3.9 | 3.5 | 2.0 |

As is clear from the results described above, the topical agents of the present invention showed an extremely high rate of preventing the edema caused by carrageenin due to the pharmacological effect of indometacin, as compared to the comparative preparation. In particular, the topical agent obtained by formulating l-O-n-dodecyl-3-O-methyl-2-O-2',3'-dihydroxypropylglycerol as a percutaneous absorption accelerator showed extremely high pharmacological effects of indometacin.

Example 3

A topical agent containing mefenamic acid shown below was prepared and the percutaneous absorption was tested. The results are shown in Table 5.

Preparation 9 of the present invention:

Mefenamic acid, 1 g, was incorporated in a mixture of 10 g of propylene glycol, 5 g of l-O-n-dodecyl-3-O-methyl-2-O-2',3'-dihydroxypropylglycerol and 30 g of ethanol. The mixture was added to a swollen mixture of 1 g of "HIVISWAKO-104®"(made by Wako Pure Chemical Industries, Ltd., carboxymethyl polymer) in 20 g of purified water. After the mixture was homogeneously mixed, 3 g of 2% ammonia water was added thereto while stirring and, purified water was further added thereto to make 100 g to obtain a gel ointment.

Comparative preparation 4

Mefenamic acid, 1 g, was incorporated in a mixture of 15 g of propylene glycol and 30 g of ethanol. The mixture was added to a swollen mixture of 1 g of "HIVISWAKO-104®"(made by Wako Pure Chemical Industries, Ltd., carboxymethyl polymer) in 20 g of purified water. After the mixture was homogeneously mixed, 3 g of 2% ammonia water was added thereto while stirring and, purified water was further added thereto to make 100 g to obtain a gel ointment.

Method:

Test for percutaneous absorption of mefenamic acid:

Seven (7) Japanese white female rabbits weighing about 3 kg were used as one group. The topical agents of the present invention and the comparative preparations were applied onto the normal abdominal skin (10 cm x 14 cm) of the rabbits in each group, from which the body hair was cut, respectively, at a dose corresponding to 50 mg of mefenamic acid. Blood was collected from the vein of the ear after 4, 10 and 20 hours and, blood concentration of mefenamic acid was measured.

Table 5

| | Concentration of Mefenamic Acid in Serum (C; µg/ml) | | |
|---|---|---|---|
| Preparation | 4 hrs | 10 hrs | 20 hrs |
| This invention 9 | 4.9 | 5.1 | 4.7 |
| Comparative Preparation | 1.8 | 1.1 | 0.4 |

As is clear from the results described above, the topical agent of the present invention showed extremely high percutaneous absorption of mefenamic acid, as compared to the comparative preparation.

Example 4

Cataplasms shown below were prepared and, the percutaneous absorption of methyl salicylate was tested. The results are shown in Table 6.

Preparation:

Preparation 10 of the present invention:

To 10 parts (weight basis, hereafter the same) of gelatin were added 35 parts of water. The mixture was warmed to 70 °C to dissolve gelatin therein. To the solution were added 12 parts of titanium oxide, 10 parts of glycerol, 10 parts of sorbitol and 10 parts of l-O-n-dodecyl-3-O-methyl-2-O-2',3'-dihydroxypropylglycerol. Then, 5 parts of self-cross linkable sodium polyacrylate (prepared in accordance with Example 1 of Published Examined Japanese Patent Application 30710/79) and 5 parts of sodium carboxymethyl cellulose were added to the mixture. Further 3 parts of a drug (drug mixture obtained by formulating l-menthol, d-camphor and methyl salicylate in a weight ratio of 5 : 1 : 4) were added thereto and, the mixture was kneaded to obtain a cataplasm paste composition. The composition was coated on a lint sheet. A polypropylene film was applied to the paste surface, which was cut into an appropriate size to obtain a cataplasm.

Preparation 11 of the present invention:

A cataplasm similar to Preparation 10 of the present invention was prepared by formulating 10 parts of l-O-methyl-branched isostearyl-3-O-methyl-2-O-2',3'-dihydroxypropylglycerol instead of l-O-n-dodecyl-3-O-methyl-2-O-2',3'-dihydroxypropylglycerol in Preparation 10 of the present invention, 10 parts of glycerol and 10 parts of sorbitol.

Comparative preparation 5

A cataplasm similar to Preparation 10 of the present invention was obtained except that 15 parts of glycerol and 15 parts of sorbitol were used in place of 10 parts of glycerol and 10 parts of l-O-n-dodecyl-3-O-methyl-2-O-2',3'-dihydroxypropylglycerol.

Method:

Test for percutaneous absorption of methyl salicylate:

Seven (7) Japanese white female rabbits weighing about 3 kg were used as one group. Each sheet of the cataplasms of the present invention and the comparative preparation, in which 1.2 wt% of methyl salicylate was formulated, were applied to the normal abdominal skin (10 cm x 14 cm) of the rabbits in each group, from which the body hair was cut, respectively. Blood was collected from the vein of the femur after 3, 6, 10, 20 and 30 hours and, blood concentration of methyl salicylate was measured.

Table 6

| Preparation | | Maximum Concentration in Serum (Cmax; μg/ml) | Reaching Time (Tmax; hr) |
|---|---|---|---|
| This invention | 10 | 47 | 10 |
| " | 11 | 41 | 10 |
| Comparative Preparation | 5 | 14 | 6 |

As is clear from the results above, the cataplasms of the present invention all showed extremely high percutaneous absorption of methyl salicylate, as compared to the cataplasm for comparison. In particular, the cataplasm of the present invention obtained by formulating l-O-n-dodecyl-3-O-methyl-2-O-2',3'-dihydroxypropylglycerol as the percutaneous absorption accelerator showed extremely high percutaneous absorption of methyl salicylate.

Example 5

Topical agents containing naphthiomate shown below were prepared and, the percutaneous absorption was tested. The results are shown in Table 7.

Preparation:

Preparation 12 of the present invention:

A mixture of 1 g of naphthiomate, 5 g of l-O-n-dodecyl-3-O-methyl-2-O-2',3'-dihydroxypropylglycerol, 14 g of glycerol triacetate, 30 g of methyl ethyl ketone and 50 g of ethanol was made a liquid topical agent.

Comparative preparation 6

A mixture of 1 g of naphthiomate, 19 g of glycerol triacetate, 30 g of methyl ethyl ketone and 50 g of ethanol was made a liquid topical agent.

Method:

Test for percutaneous absorption of naphthiomate:

Wistar-strain male 10 rats, weighing about 150 g, were used as 1 group. The topical agent was applied to the normal back skin (5 cm x 4 cm) of the rats of each group, from which the body hair was cut, in a dose corresponding to 2 mg of naphthiomate. Blood was collected from the abdominal main artery after 10 hours and, blood concentration of naphthiomate was measured.

Table 7

| Preparation | Concentration of naphthiomate in serum (C; ng/ml) |
|---|---|
| This invention 12 | 29.5 |
| Comparative Preparation 6 | 9.3 |

As is clear from the results described above, the topical agent prepared in the example showed extremely high percutaneous absorption of naphthiomate, as compared to the comparative example.

Example 6

Aspirin Suppository:

| | | |
|---|---|---|
| (1) | Pharmacopeial aspirin | 1 g |
| (2) | 1-O-n-Dodecyl-3-O-methyl-2-O-2',3'-dihydroxypropylglycerol | 0.5 g |
| (3) | Homotex®(made by Kao Soap Co., Ltd.; medium chain fatty acid triglyceride) | 8.5 g |

(1) to (3) were thoroughly agitated and mixed and, 1 g each of the mixture was filled up in a soft gelatin capsule to prepare an aspirin suppository.

The aspirin suppository of Example 6 was administered to male rabbits weight about 3 kg and, change in blood concentration of salicylic acid was measured. For control, the following was used.

Control: suppository containing 100 mg of pharmacopeial aspirin in Homotex as a base.

The results are shown in Fig. 1.

Example 7

Indometacin Suppository:

| | | |
|---|---|---|
| (1) | Pharmacopeial indometacin | 1.5 g |
| (2) | 1-O-Methyl-branched isostearyl-3-O-methyl-2-O-2',3'-dihydroxypropylglycerol | 0.5 g |
| (3) | Homotex®(supra) | 8.0 g |

(1) to (3) were thoroughly agitated and mixed and, 1 g each of the mixture was filled up in a soft gelatin capsule to prepare an indometacin suppository.

Example 8

In a manner similar to Example 6, an aspirin suppository was prepared using l-O-n-octyl-3-O-methyl-2-O-2',3'-dihydroxypropylglycerol.

Example 9

In a manner similar to Example 7, an indometacin suppository was prepared using l-O-n-dodecyl-3-O-methyl-2-O-2',3'-dihydroxypropylglycerol.

Example 10

Insulin Suppository:

| | | |
|---|---|---|
| (1) | Solution of 100 IU of insulin in 0.5 ml of a 6% aqueous acetic acid solution | 0.5 g |
| (2) | 1-O-n-Dodecyl-3-O-methyl-2-O-2',3'-dihydroxypropylglycerol | 0.5 g |
| (3) | Homotex | 9.0 g |

After (1) to (3) were thoroughly agitated to disperse, 1 g each of the dispersion was filled up in a soft gelatin capsule to prepare an insulin suppository.

Example 11

Insulin Spraying Agent for Nasal Use:

| | | |
|---|---|---|
| (1) | Solution of 1000 IU of insulin in 2.0 ml of a 6% aqueous acetic acid solution | 2 g |
| (2) | 1-O-Methyl-branched isostearyl-3-O-methyl-2-O-2',3'-dihydroxypropylglycerol | 2 g |
| (3) | Ethanol | 6 g |
| (4) | Physiological saline solution | 90 g |

(1) to (4) were thoroughly mixed and, the mixture was filled up in a pump sprayer to make an insulin spraying agent for nasal use.

Brief Description of the Drawing:

Fig. 1 shows a change in blood concentration of salicylic acid when the aspirin suppository of the present invention and the control aspirin suppository containing no transmucosal absorption accelerator were administered to rabbits.

**Claims**

1. A percutaneous absorption accelerator comprising an ether compound of a glycerol or a polyglycerol and an alcohol represented by the following formula (I) or (II):

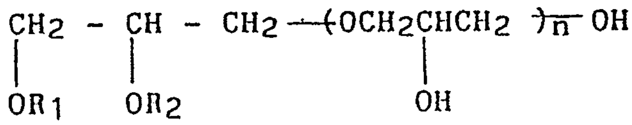

(I)

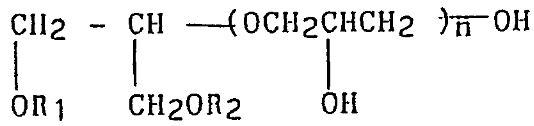

(II)

wherein $R_1$ represents a saturated or unsaturated straight or branched aliphatic hydrocarbon group having 8 to 24 carbon atoms and $R_2$ represents an alkyl group having 1 to 4 carbon atoms and n represents an integer of 1 to 10.

2. A percutaneous absorbent preparation comprising a pharmaceutically effective component and an ether compound of a glycerol or a polyglycerol and an alcohol represented by the following formula (I) or (II):

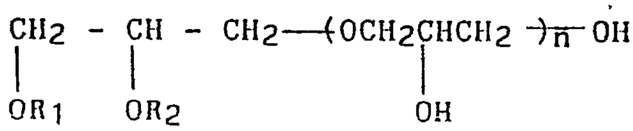

(I)

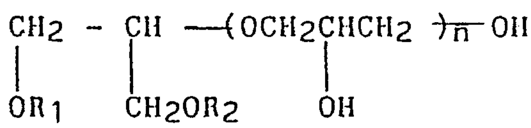

(II)

wherein $R_1$ represents a saturated or unsaturated straight or branched aliphatic hydrocarbon group having 8 to 24 carbon atoms and $R_2$ represents an alkyl group having 1 to 4 carbon atoms and n represents an integer of 1 to 10.

**Revendications**

1. Accélérateur d'absorption percutanée comprenant un éther d'un glycérol, ou d'un polyglycérol, et d'un alcool représenté par la formule générale (I) ou (II) suivante :

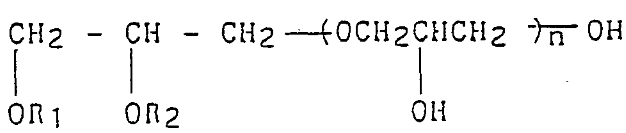

(I)

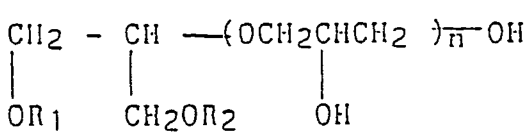

(II)

où $R_1$ représente un groupe hydrocarboné aliphatique droit ou ramifié saturé ou insaturé ayant 8 à 24 atomes de carbone, $R_2$ représente un groupe alkyle ayant 1 à 4 atomes de carbone et n représente un entier de 1 à 10.

**2.** Préparation à absorption percutanée comprenant un composant pharmaceutiquement efficace et un éther d'un glycérol, ou d'un polyglycérol, et d'un alcool représenté par la formule générale (I) ou (II) suivante :

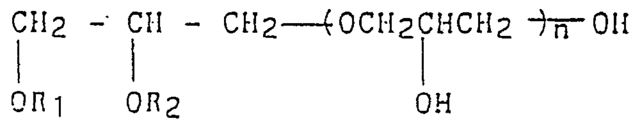

(I)

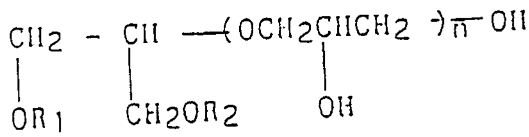

(II)

où $R_1$ représente un groupe hydrocarboné aliphatique droit ou ramifié saturé ou insaturé ayant 8 à 24 atomes de carbone, $R_2$ représente un groupe alkyle ayant 1 à 4 atomes de carbone et n représente un entier de 1 à 10.

## Patentansprüche

**1.** Perkutaner Absorptions-Beschleuniger, dadurch **gekennzeichnet**, daß er eine Etherverbindung eines Glycerins oder eines Polyglycerins und eines Alkohols der folgenden Formel (I) oder (II):

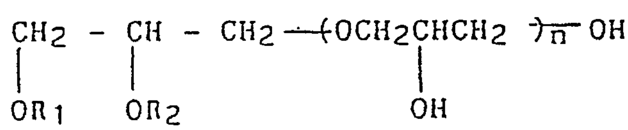

(I)

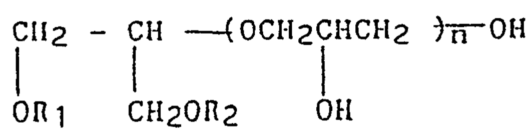

(II)

worin $R_1$ für eine gesättigte oder ungesättigte geradkettige oder verzweigte aliphatische Kohlenwasserstoffgruppe mit 8 bis 24 Kohlenstoffatomen steht und $R_2$ für eine Alkylgruppe mit 1 bis 4 Kohlenstoffatomen steht und n eine ganze Zahl von 1 bis 10 ist, enthält.

**2.** Perkutane Absorptions-Zubereitung, dadurch **gekennzeichnet**, daß sie eine pharmazeutisch wirksame Komponente und eine Etherverbindung eines Glycerins oder eines Polyglycerins und eines Alkohols der folgenden Formel (I) oder (II):

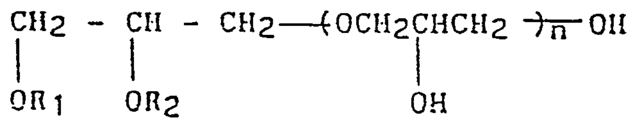

(I)

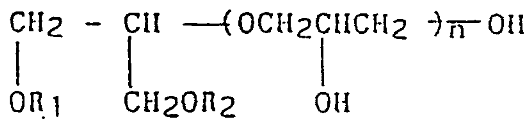

(II)

worin $R_1$ für eine gesättigte oder ungesättigte geradkettige oder verzweigte aliphatische Kohlenwasserstoffgruppe mit 8 bis 24 Kohlenstoffatomen steht und $R_2$ für eine Alkylgruppe mit 1 bis 4 Kohlenstoffatomen steht und n eine ganze Zahl von 1 bis 10 ist, enthält.

Fig. 1

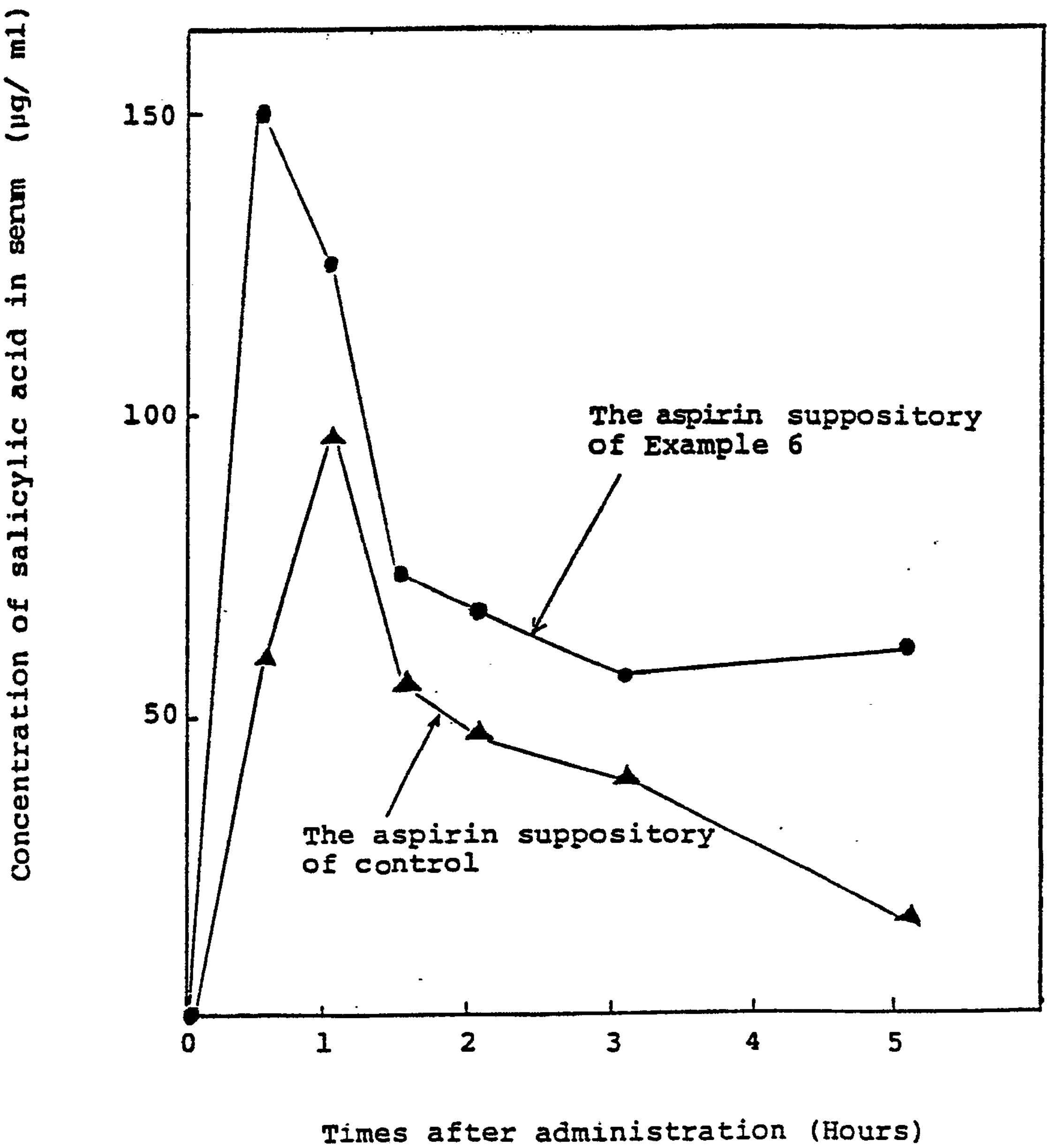
Concentration of salicylic acid in serum (μg/ ml)
150
100
50
0
1
2
3
4
5
The aspirin suppository of Example 6
The aspirin suppository of control
Times after administration (Hours)